(19) Europäisches Patentamt
European Patent Office
Office européen des brevets

(11) **EP 2 788 039 B1**

(12) **FASCICULE DE BREVET EUROPEEN**

(45) Date de publication et mention de la délivrance du brevet:
**12.07.2017 Bulletin 2017/28**

(21) Numéro de dépôt: **12810380.1**

(22) Date de dépôt: **07.12.2012**

(51) Int Cl.:
*A61L 24/06* $^{(2006.01)}$    *A61L 27/16* $^{(2006.01)}$

(86) Numéro de dépôt international:
**PCT/FR2012/052847**

(87) Numéro de publication internationale:
**WO 2013/083935 (13.06.2013 Gazette 2013/24)**

(54) **CIMENTS POLYMERES POUR LA FIXATION DE PROTHESES, LA REPARATION OSSEUSE ET LA VERTEBROPLASTIE OBTENUS A PARTIR DE FORMULATIONS MONOPHASIQUES LIQUIDES.**

AUS EINPHASIGEN FLÜSSIGEN FORMULIERUNGEN GEWONNENE POLYMERZEMENTE ZUR FIXIERUNG VON PROTHESEN, ZUR KNOCHENREPARATUR UND FÜR DIE VERTEBROPLASTIE

POLYMER CEMENTS USED FOR FIXING PROSTHESES, FOR BONE REPAIR AND FOR VERTEBROPLASTY, AND OBTAINED FROM LIQUID SINGLE-PHASE FORMULATIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.12.2011 FR 1161395**

(43) Date de publication de la demande:
**15.10.2014 Bulletin 2014/42**

(73) Titulaire: **Polymerexpert SA**
**33600 Pessac (FR)**

(72) Inventeurs:
• **DOLATKHANI, Marc**
**F-33610 Cestas (FR)**
• **HUPIN, Christophe**
**F-33770 Salles (FR)**

(74) Mandataire: **Santarelli**
**49, avenue des Champs-Elysées**
**75008 Paris (FR)**

(56) Documents cités:
WO-A1-2010/005442     WO-A2-2010/115138
WO-A2-2011/004355     US-A1- 2007 027 230
US-A1- 2010 228 358

**Description**

L'invention concerne un procédé de préparation de ciments polymères pour la chirurgie réparatrice ou de reconstruction osseuse, en particulier pour la fixation de prothèses, la réparation osseuse ou la vertébroplastie, à partir de formulations monophasiques liquides, ainsi que les ciments préparés à partir de ces formulations monophasiques. Elle concerne également un dispositif pour l'injection de ces formulations au site de traitement, ainsi qu'un procédé d'utilisation par activation/injection desdites préparations monophasiques liquides.

Les ciments polymères sont largement utilisés en chirurgie réparatrice pour la fixation de prothèses, la réparation osseuse et la vertébroplastie. Les premiers ciments pour réparation et comblement osseux sont apparus dans les années 1960 suite aux travaux de J. Chamley (J.Bone Joint Surg, 1960, 42B:28), sur l'utilisation de pâtes thermodurcissables à base de monomères acryliques, plus particulièrement de méthacrylate de méthyle (MMA), pour fixer une prothèse sur un fémur.

Cette technique a été rapidement reconnue comme la procédure standard de fixation de prothèses. Des améliorations au plan de la formulation de ces ciments ont été apportées par la suite, notamment par l'ajout de charges radio-opacifiantes permettant la visualisation du ciment pendant et après l'intervention, ou encore par l'adjonction d'antibiotiques qui sont progressivement libérés par le ciment, réduisant ainsi les risques d'infection.

A l'heure actuelle il existe environ trente marques commerciales de formulations de ciments pour fixation de prothèse et réparation osseuse approuvées par l'Agence Française de Sécurité Sanitaire des produits de santé et la Food and Drug Administration (Lewis G., J Biomed Mater Res B: Appl. Biomater. 2008, 84B:301).

On peut distinguer deux types principaux de formulations pour ciments acryliques : les formulations pour ciments à haute viscosité pour lesquels le chirurgien mélange et modèle la pâte à la main, et les formulations pour ciments à basse ou moyenne viscosité destinées à être injectées via une seringue ou un trocart, et qui sont utilisées notamment en comblement osseux et vertébroplastie Lewis G., J. Biomed. Mater Res B, 2006, Appl Biomater, 76B: 456-468. Toutes les formulations pour ciments polymères sont à base de préparations bi- ou polyphasiques, que le praticien doit mélanger manuellement juste avant utilisation. Le terme « phase » s'applique ici pour toute formulation liquide ou solide contenue et conservée indépendamment dans un réservoir, telle qu'une solution liquide, un mélange liquide ou solide, homogène ou d'apparence homogène au niveau macroscopique (suspension stable, mélange de poudres,..).

Ainsi, par « formulation bi- ou poly-phasique » ou « préparation bi- ou poly-phasique », on entend que les différents composants intervenant dans la préparation des ciments polymères; à savoir monomères, polymères, agent radio-opacifiant, amorceur, activateur ainsi que tout autre additif, sont répartis en plusieurs phases conservées dans des réservoirs séparés (au moins deux), les phases contenues dans les différents réservoirs étant mélangées pour initier la prise du ciment, juste avant ou durant l'opération d'injection. Ces formulations bi-ou poly-phasiques ont pour objectif d'éviter que les monomères soient en contact avec l'amorceur ou, pire encore, que les monomères soient en contact simultanément avec l'amorceur et l'agent d'activation, empêchant de ce fait le démarrage de la réaction de polymérisation et une prise en masse rapide et rédhibitoire des formulations.

Une revue du domaine indique que, encore aujourd'hui, la quasi-totalité des ciments commerciaux se présente sous la forme d'une phase solide en poudre et d'une phase liquide à mélanger (Osteopal-V®, Osteo-Firm®, Vertebroplastic®, Simplex®, Cranioplastic®, Palacos®, CMV®, KyphX HV-R® pour les principaux).

La phase solide contient principalement des billes de poly(méthacrylate de méthyle) (PMMA) ou de copolymères du PMMA (co-PMMA), un amorceur (initiateur) de polymérisation radicalaire, en général du peroxyde de benzoyle (BPO), et une charge radio-opacifiante, telle que du sulfate de baryum ($BaSO_4$) ou du dioxyde de zirconium ($ZrO_2$). La phase liquide est composée de(s) monomère(s), tel(s) que par exemple le méthacrylate de méthyle (MMA), éventuellement en mélange avec des co-monomères, et d'un activateur radicalaire, en général de la N,N-diméthyl-*p*-toluidine (DMPT) lorsque du BPO est utilisé dans la phase solide (Mc Graw JK et al. 2003, J Vasc Interv Radiol;14:S311-5).

Dans ces formulations bi-phasiques, les monomères ne sont donc pas en contact avec l'amorceur (ou initiateur), ni avec l'association amorceur/activateur pendant la phase de stockage, le mélange se faisant au moment de l'application.

Ces formulations biphasiques, constituées par une phase liquide et une phase solide que l'on mélange pour générer le ciment juste avant son injection, sont dénommées dans le présent document « formulation standard».

L'application de ces ciments, d'abord conçus pour la fixation de prothèses, à la vertébroplastie a été réalisée en 1985 (Galibert P et al., 1987, Neurochirurgie, 33:166. Le principe vise à introduire dans une vertèbre endommagée la préparation de ciment qui, en durcissant *in situ*, va constituer un renfort mécanique en comblant la cavité. Les indications pour cette intervention sont, notamment, les angiomes vertébraux, les métastases rachidiennes entraînant des fractures vertébrales et les tassements ostéoporotiques qui affectent particulièrement les femmes et les hommes âgés.

Avec le vieillissement de la population la vertébroplastie est en plein essor. Le nombre d'interventions est estimé à 2 millions par an pour un marché de 3 milliards de dollars. Rien qu'aux Etats-Unis et en Europe, 700 000 et 450 000 cas cliniques de fracture vertébrale sont respectivement détectés par an.

La technique de chirurgie proprement dite consiste à injecter par voie percutanée et sous contrôle radiologique, un ciment polymère de basse ou moyenne viscosité dans une vertèbre pathologique pour obtenir une consolidation de cette vertèbre. Pour cette intervention, le praticien opère généralement sous anesthésie locale en perforant le corps vertébral à l'aide d'un trocart et suit l'intervention sous scopie. Une fois le trocart idéalement placé, un ciment acrylique radio-opaque, du même type que ceux utilisés pour les opérations visant la hanche ou les genoux, est préparé en mélangeant les différents composants, puis injecté de manière à remplir le corps vertébral. Le ciment, en durcissant par polymérisation, vient ainsi renforcer la vertèbre.

Dans le cas de la plupart des ciments commerciaux, pour pouvoir réaliser la vertébroplastie, le praticien doit dans une première phase manuelle procéder au mélange des différentes formulations contenant les composants du ciment sous agitation vigoureuse, jusqu'au début de prise du ciment caractérisé par une augmentation rapide de sa viscosité et de sa température. Cette phase préliminaire, d'une part, expose les praticiens aux vapeurs toxiques de monomère et d'agent activateur (DMPT) et, d'autre part, constitue, en raison de son caractère manuel, une cause avérée d'inhomogénéité dans les propriétés des ciments obtenus, notamment en raison de l'inclusion de bulles d'air formées sous agitation. Ces bulles vont provoquer après polymérisation l'apparition de pores, hétérogènes en taille, et conduire à des zones de fragilité dans le matériau.

La durée de cette phase initiale de polymérisation dépend de la composition des formulations, de la température ambiante mais également du mode d'agitation et de mélange des différentes phases qui varient avec chaque manipulateur. Cette étape préliminaire, réalisée en présence de l'oxygène de l'air est également susceptible de provoquer une désactivation partielle plus ou moins importante des radicaux initiant la réaction de polymérisation. Les aléas liés à cette phase de mélange manuel sous air ont été réduits grâce à la méthode qui consiste à centrifuger/mélanger mécaniquement, sous vide modéré le mélange poudre-liquide, mais cette voie requiert un équipement spécial en salle d'opération (Wixson, R.L. et al. J. of Arthroplasty, 1987, Vol 2, Issue 2, 141-149).

La fin de cette phase initiale est définie empiriquement comme étant le moment où le ciment ne colle plus aux gants du praticien. Le temps « de travail » pendant lequel le ciment est ensuite utilisable, c'est-à-dire introduit dans la seringue puis injecté via le trocart, est limité à quelques minutes (typiquement 8 à 15 minutes). Un ciment dont la prise est trop avancée risque de bloquer le trocart, tandis qu'un ciment trop fluide (contenant une fraction importante de monomère toxique, non encore polymérisé) présente le risque de s'écouler hors de la vertèbre, causant des complications post-opératoires importantes (compression veineuse, fuite péridurale, hypotension artérielle,..). Ces risques peuvent être réduits en passant au préalable dans la vertèbre un ballon qui va être gonflé *in situ* pour redresser la vertèbre et créer une cavité plus étanche dans laquelle est introduit le ciment. On parle alors de kyphoplastie, mais cela rend l'intervention plus complexe, plus lourde et plus coûteuse.

D'autres approches ont été proposées afin d'améliorer les procédures et de réduire les risques opératoires.

La demande de brevet WO2010/005442 concerne des préparations de ciments « multi-solutions » ou encore multi-phasiques liquide/liquide constituées de deux formulations (phases) liquides. Les formulations liquides décrites sont constituées par des solutions (ou plus précisément des suspensions, dans de nombreux cas), à base de monomère (MMA) contenant des chaînes de polymère (PMMA) sous diverses formes (linéaires, particules réticulées, peignes), un agent radio-opacifiant ; l'une des formulations contenant en plus l'amorceur (BPO) et l'autre l'activateur (DMPT). Ces deux formulations sont disposées chacune dans une chambre d'un injecteur à deux chambres, équipé en sortie d'un mélangeur dans lequel les deux formulations liquides sont mises en contact et mélangées au moment de l'opération d'injection. Le démarrage de la polymérisation du MMA qui s'ensuit résulte de l'action combinée du BPO et de la DMPT sur le(s) monomère(s).

Un défaut majeur de cette méthode consiste en la stabilité réduite de la solution de MMA contenant le BPO, dont la décomposition en radicaux amorceurs intervient de façon lente mais continue, même à des températures de 10°C, voire à des températures inférieures, comme rapporté par Sivaram, S. et al., Polymer Bulletin, 1980, 3, 1-2, , 27-35. Ceci limite la conservation et l'utilisation dans le temps de ces formulations liquides dont la viscosité augmente même lorsqu'elles sont conservées au réfrigérateur (4°C). Par ailleurs, il est notoire que l'injection à l'aide d'une seringue bi-compartiment de deux phases possédant des viscosités différentes est difficilement maîtrisable et présente des risques d'inhomogénéité dans la répartition des composants des deux phases lors du mélange, inhomogénéité qui se répercute sur les propriétés finales des ciments.

Avec le même objectif, à savoir d'automatiser le procédé et de réduire les opérations manuelles, la demande US 2007/0027230 décrit des dispositifs permettant la préparation, le mélangeage et l'injection dans la vertèbre du patient de ciments à haute viscosité (typiquement supérieure à 500 Pa.s et pouvant atteindre 2000 Pa.s) après une période allant de quelques minutes à une dizaine de minutes). Les dispositifs d'injection décrits ont la capacité d'appliquer des pressions très élevées qui permettent de préparer et d'introduire des ciments de haute viscosité jusqu'à la vertèbre à traiter. Des compositions biphasiques ou monophasiques pour ciments à haute viscosité ayant des vitesses de durcissement et des caractéristiques de dureté diverses sont revendiquées.

Concernant les ciments constitués à partir de deux phases distinctes, l'une liquide contenant monomère, activateur et divers additifs, l'autre solide contenant polymère, amorceur et d'autres additifs, ils correspondent à des formulations « standard » car conduisant à des ciments à haute viscosité.

Les formulations monophasiques décrites dans la demande US 2007/0027230 sont constituées par une phase unique contenant un ou des polymères sans aucun composant monomère. Les ciments à « haute viscosité » correspondants ne font appel à aucune étape de polymérisation et leur « durcissement » repose uniquement sur le changement des propriétés thermomécaniques des polymères avec la température. Ces formulations « tout polymère » sont constituées d'un polymère ou d'un mélange de polymères ayant des températures de ramollissement (Tg) légèrement supérieures à la température corporelle. Ils sont injectés à une température supérieure à la température de ramollissement à l'aide d'un dispositif permettant d'appliquer de fortes pressions. Ces ciments de très haute viscosité sont dits « non-durcissants ».

Cette stratégie, basée sur la conception de dispositifs d'injection « haute pression » intégrant ou non les étapes de préparation/mélange de ciments à « haute viscosité » permet l'absence ou l'utilisation d'une quantité réduite de monomère toxique, ce qui limite les risques inhérents à son introduction dans le corps du patient. Cette approche permet également une limitation voire une suppression du pic de température qui caractérise la phase de polymérisation (Tpic > 80°C dans certains procédés), évitant les risques de nécrose des tissus à proximité. Cependant, cette voie nécessite l'application de pressions très élevées (200 atmosphères, voire supérieures), ce qui constitue un risque important d'endommagement et/ou de rupture des vertèbres ou des os à consolider, qui sont dans un état fragilisé.

Un autre problème, rencontré en particulier en réparation osseuse et en vertébroplastie, concerne la dureté trop élevée et la faible compressibilité et flexibilité des ciments commerciaux actuels, ce qui peut entraîner de nouvelles fractures osseuses par transmission des contraintes mécaniques. Ceci est relativement fréquent au niveau de la vertèbre réparée mais également au niveau des vertèbres voisines, sur lesquelles les contraintes sont directement transférées sans amortissement. Ainsi Grados et al. (Rheumatology 2000 ; 39 : 1410-4) ont rapporté, dans un suivi moyen de quatre ans, que le risque relatif de fracture vertébrale au voisinage d'une vertèbre tassée passait de 1,44 à 2,27 après une vertébroplastie.

Dans un même ordre d'idée, Cyteval et al. (AJR 1999; 173 : 1685-90) ont mis en évidence de nouvelles fractures vertébrales dans 25 % des cas à l'issue de six mois suivant les fractures traitées. Pour tenter de remédier à ce problème des ciments présentant des caractéristiques de dureté et de compressibilité appropriées aux propriétés de la vertèbre ou de tout autre élément à traiter ont été développés.

Ainsi la demande US2007/0027230 citée précédemment, mais également les demandes WO2010/115138, WO2011/004355 et US2010/0228358 proposent des formulations particulières de ciments intégrant, respectivement, soit des polymères particuliers, tels que des PMMA modifiés apportant une porosité, soit des particules de polymères réticulées hydrophiles, soit encore des particules de verre ou de céramiques afin de moduler les propriétés de dureté et de compressibilté des ciments. Sauf exception, la préparation de ces ciments est réalisée par mélangeage manuel d'une phase liquide et d'une phase solide.

Il n'existe donc pas, à ce jour, de méthodes totalement satisfaisantes de préparation et de mise en oeuvre de formulations conduisant à des ciments acryliques présentant des propriétés optimales pour la vertébroplastie. Il n'existe pas, en particulier, de procédé basé sur la simple mise en oeuvre de préparations monophasiques pour ciments polymères excluant toute opération complexe de mélangeage entre phases.

Des innovations telles que celles apportées par l'invention, permettent de simplifier et de maîtriser les étapes de préparation et d'injection des ciments polymères.

Ainsi, l'invention fait appel à l'utilisation d'une formulation monophasique à base de monomère(s) contenant le ou les polymères ou (co)polymère(s) dissous et/ou en suspension, un agent activateur de polymérisation, et éventuellement au moins un agent radioopacifiant et divers autres additifs.

Par « formulation monophasique », on entend que cette formulation, qui contient plusieurs composants (en solution ou en suspension), est contenue dans un réservoir unique.

Par « liquide », on entend que cette formulation peut être sous forme de solution ou de suspension ayant une viscosité telle qu'elle peut être injectée par le biais d'un dispositif d'injection, par exemple de type seringue, utilisé de manière usuelle dans le domaine de la vertébroplastie, sans nécessiter l'application d'une pression importante susceptible d'endommager la vertèbre ou l'os.

La formulation monophasique liquide selon l'invention est conservée dans le réservoir d'un dispositif d'injection étanche et la polymérisation est activée lors de l'opération d'injection par passage sur une couche mince ou un film de l'amorceur déposé sur un élément du dispositif. Outre sa facilité d'application, ladite formulation, une fois passée sur le film d'amorceur ne requiert aucun temps d'attente particulier avant l'injection dans la vertèbre ou l'os, le film polymère contenant l'amorceur se solubilisant immédiatement au passage de la formulation monophasique. Ceci représente un avantage majeur par rapport aux procédés bi- ou multiphasiques dans lesquels le polymère sous forme de particules solides doit être mélangé avec la phase monomère, puis solubilisé, ce qui implique un temps d'attente plus ou moins long, à l'appréciation du praticien, avant son injection.

De plus, la stabilité dans le temps de la formulation monophasique liquide selon l'invention, conservée en l'absence de l'amorceur, est également un avantage important vis à vis des formulations liquides/liquides dans lesquelles l'une des phases contenant le monomère est en contact avec l'amorceur, ce qui induit un risque important de polymérisation.

Comparée aux formulations monophasiques non durcissables, basées sur l'injection, à haute température et à des pressions élevées, de compositions à l'état fondu (US 2007/0027230), les formulations selon l'invention présentent l'avantage d'une mise en oeuvre à température ambiante et à des pressions ne présentant pas de risque d'endommagement des vertèbres.

Enfin, l'invention concerne l'utilisation de systèmes (co)polymères particuliers conduisant à des ciments possédant une phase souple élastomère dispersée dans une matrice polymère plus rigide, les ciments ainsi obtenus présentant des propriétés de résistance mécanique et de compressibilité appropriées à la zone à consolider et permettant de limiter les risques de propagation de contraintes élevées et de ruptures osseuses.

Les bénéfices apportées par ce procédé nouveau pour la fixation de prothèses, la réparation osseuse et la vertébroplastie sont très attendus par les praticiens et amélioreront les traitements des patients.

La présente invention concerne un procédé de préparation de ciments polymères pour la chirurgie réparatrice ou de reconstruction osseuse, en particulier pour la fixation de prothèses, la réparation osseuse et la vertébroplastie, à partir de formulations monophasiques liquides particulières.

Ces ciments peuvent être utilisés dans les domaines de la chirurgie réparatrice ou de reconstruction, ou en chirurgie esthétique, en chirurgie humaine ou vétérinaire.

L'invention concerne également les ciments particuliers obtenus par ce procédé, ainsi qu'un procédé d'activation/injection spécifique de ces formulations et, un dispositif d'application dédié.

Les formulations monophasiques liquides comprennent des solutions ou suspensions de polymères ou de copolymères, d'activateur(s) de polymérisation, d'agent(s) radio-opacifiant(s) le cas échéant, et éventuellement d'additifs (antibiotique, inhibiteur...) dans des monomères, de préférence des monomères acryliques.

Le dispositif d'injection, qui est également un objet de l'invention comprend un injecteur mono-compartiment (contenant la formulation monophasique) équipé en sortie d'un élément de support, tel que par exemple un mélangeur statique, sur lequel est déposé(e) une couche mince ou un film contenant l'amorceur de polymérisation, ou encore une aiguille ou une canule à l'intérieur de laquelle est déposé(e) une couche mince ou un film contenant l'amorceur de polymérisation. Lors de l'opération d'injection, la formulation monophasique liquide est mise en contact avec l'amorceur par passage sur le film contenant l'amorceur, entraînant ainsi sa solubilisation et mettant celui-ci en contact avec l'activateur et le(s) monomère(s), ce qui induit le démarrage de la polymérisation. Un trocart fixé(e) à l'extrémité du mélangeur statique permet d'introduire la formulation directement dans la cavité osseuse où doit être fixée la prothèse ou dans l'os ou la vertèbre à consolider, évitant tout mélangeage manuel, toute opération de débullage et tout contact de la formulation avec l'environnement extérieur et les risques qui y sont associés.

L'invention concerne donc un procédé de préparation et de mise en oeuvre de ciments biomédicaux injectables basé sur l'utilisation de formulations monophasiques, ainsi que les ciments particuliers qui en sont issus, et le dispositif permettant leur injection.

Ledit procédé comprend la mise en contact d'une formulation monophasique liquide, ladite formulation comprenant une phase liquide contenant au moins un polymère ou copolymère en solution ou en suspension dans au moins un monomère, et au moins un activateur de polymérisation radicalaire, avec un amorceur de polymérisation radicalaire déposé sous forme de couche mince ou de film sur un support.

De préférence, ladite formulation monophasique liquide contient également au moins un agent radioopacifiant.

Avantageusement, la mise en contact de ladite formulation monophasique liquide, avec un amorceur de polymérisation radicalaire déposé sous forme de couche mince ou de film sur un support, permet la solubilisation de ladite couche mince ou film contenant l'amorceur, la mise en contact de l'amorceur et de l'activateur de polymérisation et l'amorçage de la polymérisation du ou des monomères.

En particulier, ladite mise en contact est effectuée par le passage de ladite formulation monophasique liquide sur un élément comprenant un support sur lequel est déposé(e) une couche mince ou un film contenant un amorceur de polymérisation radicalaire.

Ainsi, la couche mince ou le film contenant l'amorceur de polymérisation radicalaire se solubilise au passage de la formulation monophasique et permet la dissolution rapide de l'amorceur de polymérisation radicalaire ainsi que sa distribution au sein de la formulation monophasique liquide.

De manière avantageuse, la mise en contact de ladite formulation monophasique liquide, avec un amorceur de polymérisation radicalaire déposé sous forme de couche mince ou de film sur un support a lieu au moment de l'intervention chirurgicale.

Avantageusement, ladite mise en contact de la formulation monophasique liquide avec la couche mince ou le film contenant l'amorceur est réalisée dans un dispositif d'injection dans lequel ladite formulation monophasique liquide est contenue dans le réservoir unique du dispositif d'injection (injecteur mono-compartiment) équipé en sortie d'un élément comprenant un support sur lequel est déposé(e) une couche mince ou un film de l'amorceur de polymérisation. Lors de l'opération d'injection, la formulation monophasique liquide est mise en contact avec l'amorceur par passage sur le film contenant l'amorceur.

Selon une alternative, le procédé selon l'invention comprend une étape de pré-polymérisation de la formulation monophasique dans un élément d'un dispositif d'injection comprenant un support sur lequel est déposé(e) une couche mince (ou film) contenant un amorceur de polymérisation, réalisée par activation de l'amorceur de polymérisation par l'activateur contenu dans la formulation monophasique. Selon l'invention, les formulations monophasiques peuvent comprendre des solutions ou suspensions liquides de monomères acryliques, contenant des polymères ou copolymères, un ou plusieurs agents radio-opacifiant(s), un ou plusieurs activateur(s) de polymérisation radicalaire et éventuellement un ou plusieurs additif(s) (antibiotique, inhibiteur de radicaux libres,..), l'ensemble constituant la monophase.

Avantageusement, la formulation monophasique liquide selon l'invention contient au moins un monomère acrylique, de préférence (méth)acrylique, un ou plusieurs polymères ou copolymères, au moins un activateur de polymérisation radicalaire, au moins un agent radio-opacifiant et au moins un additif.

De préférence, lesdites formulations sont constituées des solutions ou suspensions définies ci-dessus.

Avantageusement, ces formulations liquides monophasiques présentent une bonne stabilité dans le temps, et ne subissent aucune altération notable jusqu'à ce que le processus de polymérisation des monomères qu'elles contiennent soit volontairement déclenché par le praticien au moment de l'opération chirurgicale nécessitant l'injection du ciment. Ceci implique en particulier qu'il n'y ait pas de polymérisation spontanée des monomères au sein de la formulation. Ceci est réalisé en empêchant tout contact entre le(s) monomère(s) présent(s) et un quelconque composé générateur de radicaux amorçant, ou en bloquant les radicaux qui pourraient se former par exemple par ajout d'une quantité appropriée d'un agent inhibiteur.

Le monomère sous forme liquide dans lequel les polymères ou copolymères sont mis en solution -ou en suspension- pour former la formulation monophasique peut-être un dérivé acrylique, tel le méthacrylate de méthyle (MMA) pur ou un mélange de monomères comme du MMA avec des co-monomères de type acrylates ou méthacrylates, par exemple le (méth)acrylate de butyle (MABu) ou tout autre monomère acrylique ou méthacrylique par exemple le (méth)acrylate d'éthyle ou le (méth)acrylate de 2-éthyl hexyle ou encore un monomère non-acrylique présentant une ou plusieurs insaturations susceptibles de se polymériser par voie radicalaire.

L'ajustement du ratio quantité de monomère / quantité de polymères permet d'optimiser la viscosité de la formulation assurant une injection à des pressions sans risque d'endommagement pour les vertèbres ou l'os.

De préférence, ce ratio massique est compris entre 20 et 75%.

L'incorporation de co-monomères dans les chaînes de PMMA en formation permet de moduler les propriétés physiques des polymères (température de transition vitreuse (Tg)) et d'optimiser ainsi les propriétés mécaniques des ciments (compression, traction, cisaillement,...) afin de les adapter au mieux à l'intervention médicale et éviter les éventuels problèmes mécaniques post-opératoires. L'introduction de motifs acryliques comme les acrylates d'alkyle (par exemple en $C_2$ à $C_{10}$ comme l'acrylate de butyle ou l'acrylate d'éthyl-2 hexyle) permet ainsi d'abaisser la Tg du polymère tandis que la présence d'unités méthacrylates plus rigides (isobornyle, tulipaline par exemple) conduit à une augmentation de la Tg de la chaîne polymère.

De façon avantageuse, la présence de séquences de basse Tg dans les polymères a pour conséquence de modifier les propriétés mécaniques des ciments au voisinage de la température corporelle et notamment d'apporter un caractère amortissant du ciment vis-à-vis d'une contrainte appliquée (compression, traction, cisaillement).

Dans un aspect préféré, en vue de renforcer la cohésion au sein du ciment issu de ces formulations monophasiques, l'introduction d'un certain taux, de préférence entre 0,1 et 10 pourcents molaires, de monomères difonctionnels peut être également réalisé. On utilisera par exemple l'éthylène glycol diméthacrylate ou tout autre monomère difonctionnel polymérisable par voie radicalaire.

Les polymères qui sont mis en solution -ou en suspension- dans les monomères liquides peuvent être des polymères ou des copolymères de type statistique ou de type à blocs, en particulier du PMMA ou des copolymères statistiques ou des copolymères à blocs à base de MMA et de co-monomères tels que acrylates, méthacrylates ou tels que des monomères non-acryliques (styrène par exemple). Les chaînes de polymères ou de copolymères peuvent présenter une structure linéaire ou ramifiée ou encore former des particules constituées d'un ensemble de chaînes réticulées entre elles ou non réticulées.

Selon un aspect de l'invention, les polymères mis en solution ou en suspension dans le monomère sont choisis parmi ceux ayant une Tg basse, et ayant une nature différente des monomères. Par exemple, des polymères acrylates d'alkyles peuvent être mis en solution dans du MMA de façon à obtenir, après polymérisation, un ciment composite constitué de deux types de microdomaines, l'un ayant une Tg élevée, l'autre ayant une Tg basse, ce qui confère au matériau des propriétés amortissantes.

Selon une alternative, les polymères mis en solution ou en suspension dans le monomère peuvent être prétraités thermiquement à 120°C pendant plusieurs heures, notamment de 2 à 96 heures, avant d'être introduits dans la formulation. Ce pré-traitement thermique permet de décomposer l'amorceur résiduel contenu dans les polymères utilisés et de prévenir une polymérisation éventuelle lors de la phase de stockage en présence du mélange de monomères.

L'avantage principal de l'utilisation des copolymères statistiques consiste à pouvoir moduler les propriétés physiques et mécaniques des ciments obtenus, comme cela est explicité précédemment. Un avantage de l'utilisation des structures ramifiées ou des particules est d'obtenir des formulations de plus faible viscosité qu'en présence d'un (co)polymère linéaire de même masse molaire.

Dans une autre composition de formulation monophasique particulière, l'emploi de copolymères de type à blocs constitués de blocs non compatibles rigide/souple est une autre possibilité avantageuse de la présente invention. L'emploi de copolymères à blocs dont au moins un bloc est non compatible avec la matrice polymère formée par polymérisation des monomères de la phase liquide permet également de générer dans la matrice des micro-domaines constitués par assemblage des blocs non miscibles, les blocs miscibles assurant, eux, la cohésion avec la matrice. Cette stratégie permet en particulier d'introduire des micro-domaines constitués de blocs ayant une température de transition vitreuse basse, par exemple inférieure à 35°C, notamment inférieure à la température corporelle, et donc relativement souple, qui vont renforcer la résistance au choc et la ténacité des ciments implantés. L'exemple le plus connu de tels matériaux « choc » basé sur cette stratégie est celui du polystyrène « choc » constitué de micro-domaines de polybutadiène dispersés dans la matrice polystyrène. Cette morphologie permet d'absorber les chocs et les contraintes et de limiter la propagation de fractures dans le matériau.

A titre d'exemple, dans le cas des formulations monophasiques, les blocs souples ayant une basse Tg peuvent être constitués de divers motifs acrylate d'alkyle dont les températures de transition vitreuse varient entre +35°C et -70°C, tandis que le bloc composé de motifs méthacrylate miscible assure la cohésion avec la matrice, celle-ci apportant les caractères de rigidité et de tenue en compression.

Dans une autre formulation monophasique selon l'invention, un résultat relativement similaire peut être obtenu à partir de mélanges de PMMA avec des polymères ou copolymères non miscibles ayant une basse Tg, inférieure à 35 °C, notamment inférieure à la température corporelle, dans les monomères liquides. A titre d'exemples, on peut citer comme polymères ayant une basse Tg : les polyacrylates, le polybutadiène, les polybutadiènes hydrogénés, le polyisobutène, le polypropylène amorphe etc.

Par ailleurs, tout en conservant les caractéristiques des précédentes préparations, l'utilisation de dispersions constituées par des particules polymères ou par des particules de copolymères, non solubles dans les monomères liquides, permet d'obtenir des formulations de plus faible viscosité. Il peut être préférable dans le cas de ces préparations de réaliser avant leur utilisation une homogénéisation de la formulation par agitation.

Dans un objectif de renforcement de la cohésion au sein du ciment issu de ces formulations monophasiques, l'introduction sur les chaînes polymères de groupes fonctionnels réactifs peut être avantageusement réalisée. En permettant le couplage chimique entre chaînes, voire leur réticulation, ces groupes fonctionnels renforcent la cohésion entre les différentes phases du matériau lors de la prise du ciment. La fonctionnalité moyenne de ces formulations est choisie, de préférence, dans un domaine permettant l'avancement de la polymérisation jusqu'à des taux élevés permettant leur injection avant l'approche du point de gel caractérisé par une augmentation importante de la viscosité. Pour cela, la fonctionnalité moyenne de ces formulations est comprise entre 2 et 3 et de préférence entre 2 et 2,3, ce qui correspond dans ce dernier cas à un point de gel au-delà de 85% de conversion.

A ces solutions ou dispersions monomère/polymère peuvent être ajoutés un ou plusieurs agent(s) radio-opacifiant(s) et un ou plusieurs activateur(s) de polymérisation radicalaire. L'agent radio-opacifiant peut être introduit dans la formulation dans des proportions massiques comprises entre 30 et 60% massique et de préférence entre 40 et 50 % massique. L'agent radio-opacifiant peut être choisi, par exemple, parmi le sulfate de Baryum ou de l'oxyde de Zirconium, introduit sous forme de particules. De préférence, le diamètre moyen desdites particules est inférieur à 1 mm et de préférence inférieur à 1 μm.

L'activateur de polymérisation peut être choisi parmi les activateurs de polymérisation radicalaire. Dans le cas où l'amorceur est le peroxyde de benzoyle (BPO), on utilise de préférence la N,N-diméthyl-p-toluidine (DMPT) qui facilite la décomposition de l'amorceur dès la température ambiante, autour de 20°C. D'autres couples amorceur/activateur usuels dans le domaine, tels que, par exemple BPO/ Leuco- Crystal Violet, peroxyde de dicumyle/DMPT peuvent être également utilisés en fonction des caractéristiques recherchées pour la cinétique de polymérisation.

L'ajout d'additifs spécifiques à ces formulations monophasiques en vue d'apporter une caractéristique ou amélioration particulière à la formulation ou au ciment final peut également être réalisé. On citera, à titre d'exemple non limitatif, l'ajout d'antibiotique permettant de limiter le risque d'infection post-opératoire.

Dans certaines des formulations, l'ajout d'un agent inhibiteur de radicaux, susceptible de bloquer notamment l'action de radicaux formés *in situ* par voie thermique ou photo-chimique peut être effectué pour accroître la longévité des formulations monophasiques. On utilise dans ce cas, de préférence, des dérivés phénoliques, comme l'hydroquinone (HQ) et ses dérivés notamment. Ceux-ci sont ajoutés à des teneurs de 0 à 1000 ppm, de préférence entre 100 et 300 ppm, par rapport à l'ensemble des monomères.

Selon l'invention, le ciment polymère est préparé par un procédé comprenant la mise en contact d'une formulation monophasique telle que décrite ci-dessus avec un amorceur radicalaire sous forme de couche mince (ou film) déposée sur un support. La mise en contact de la formulation monophasique liquide avec la couche mince (ou film) contenant l'amorceur solubilise ledit amorceur et le met en contact avec le(s) activateur(s) et le(s) monomère(s) présents dans ladite formulation liquide, ce qui induit le démarrage de la polymérisation.

Avantageusement, ladite mise en contact inter faciale (passage de la formulation sur le support revêtu) solubilise le film contenant l'amorceur ce qui permet, d'une part, une répartition homogène de l'amorceur dans la formulation et, d'autre part, évite au praticien toute attente au moment de l'intervention. Ceci est un avantage majeur par rapport aux formulations biphasiques solide/liquide pour lesquelles une phase d'attente correspondant à la solubilisation du polymère dans le monomère est imposée.

La couche mince (ou film) contenant l'amorceur peut être réalisée par dépôt sur le support de solutions contenant l'amorceur, tel que, par exemple, du BPO, dans un solvant organique, qui est ensuite éliminé par évaporation. Le solvant est choisi, de préférence, parmi les composés organiques volatils non-toxiques, par exemple l'acétone ou ses homologues supérieurs, tels que, par exemple, la butanone, la diméthylcétone ou la diéthylcétone. Par « couche mince », on entend une couche d'environ 5 à 500 $\mu$m ; la quantité d'amorceur nécessaire ainsi que la surface spécifique du support conditionnant l'épaisseur de la couche.

Alternativement, l'amorceur peut être solubilisé avec un polymère filmifiant tel que, par exemple, du PMMA ou un de ses copolymères, de façon à obtenir, après dépôt sur le support et évaporation du solvant, un film de polymère contenant l'amorceur. Les caractéristiques et la proportion du polymère, tel que, par exemple, le PMMA ou un de ses copolymères, permettent de réguler la quantité d'amorceur déposé et sa vitesse de solubilisation dans la formulation monophasique.

L'invention concerne également un dispositif pour l'injection d'un ciment polymère pour la chirurgie réparatrice ou la reconstruction osseuse, en particulier pour la fixation de prothèses, la réparation osseuse et la vertébroplastie, comprenant

- un système d'injection à un compartiment, tel qu'une seringue mono-compartiment, contenant la formulation monophasique liquide décrite ci-dessus,
- un élément comprenant un support sur lequel est déposé(e) une couche mince (ou film) contenant un amorceur de polymérisation, tel que par exemple un mélangeur statique ou bien un support dont la surface spécifique est suffisante pour y déposer une couche mince (ou film) contenant l'amorceur.

Un tel support peut être, par exemple, un mélangeur statique à ailettes.

Ce dispositif peut être connecté, au moment de l'utilisation, à un système injecteur permettant d'injecter directement la formulation pré-polymérisée, en cours de prise, au site de traitement, en particulier dans la cavité osseuse ou la vertèbre, tel qu'un embout simple, une canule ou un trocart, selon l'utilisation envisagée.

Le système d'injection à un compartiment contenant la formulation monophasique liquide et l'élément comprenant un support sur lequel est déposé(e) une couche mince (ou film) contenant un amorceur de polymérisation, par exemple un système de mélange statique, et, de manière optionnelle, le système injecteur, peuvent être conditionnés séparément, notamment au sein d'un même emballage.

L'invention concerne également l'utilisation dudit dispositif pour l'injection d'un ciment polymère pour la chirurgie réparatrice ou la reconstruction osseuse, en particulier pour la fixation de prothèses, la réparation osseuse et la vertébroplastie, directement au site de traitement, en particulier dans la cavité osseuse ou la vertèbre. Ce dispositif permet d'éviter tout mélangeage manuel et tout contact de la préparation avec l'environnement extérieur et réduit significativement les risques correspondants. Il permet également de réaliser l'injection sans période d'attente correspondant à la solubilisation/dispersion d'une première phase dans une seconde phase.

L'invention concerne également un procédé d'injection d'un ciment polymère pour la chirurgie réparatrice ou la reconstruction osseuse, ledit ciment étant préparé par la mise en contact d'une formulation monophasique liquide, ladite formulation comprenant une phase liquide contenant au moins un polymère ou copolymère en solution ou en suspension dans au moins un monomère, au moins un activateur de polymérisation radicalaire, et éventuellement au moins un agent radio-opacifiant, telle que décrite plus haut, avec un amorceur de polymérisation radicalaire déposé sous forme de couche mince ou de film sur un support, tel que décrit plus haut.

L'invention concerne également un procédé d'injection d'un ciment polymère pour la chirurgie réparatrice ou la reconstruction osseuse, dans lequel on utilise un dispositif tel que décrit plus haut pour l'injection d'un ciment polymère pour la chirurgie réparatrice ou la reconstruction osseuse, en particulier pour la fixation de prothèses, la réparation osseuse et la vertébroplastie.

L'ensemble comprenant un système d'injection à un compartiment contenant la formulation liquide monophasique décrite ci-dessus, et un élément comprenant un support sur lequel est déposé(e) la couche mince (ou film) contenant l'amorceur (et, de manière optionnelle, le système injecteur) peut être conservé sous blister, à l'abri de la lumière à basse température, jusqu'à utilisation. Chaque élément de l'ensemble (système d'injection à un compartiment contenant la formulation liquide monophasique, système de mélange statique comprenant un support sur lequel est déposé(e) la couche mince (ou film) contenant l'amorceur, injecteur) peut être conditionné séparément, en vue d'une utilisation conjointe, lesdits éléments pouvant être assemblés au moment de l'injection.

Stocké à 20°C dans le noir, le film contenant un amorceur radicalaire, en particulier le BPO, peut être déposé sur les parois d'un élément du dispositif, par exemple un mélangeur statique, notamment un mélangeur statique à ailettes, et peut conserver son activité pendant plus de deux ans.

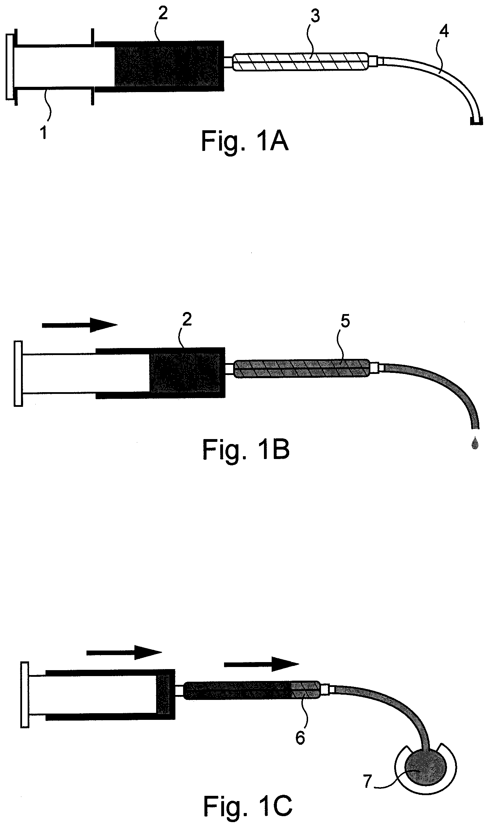

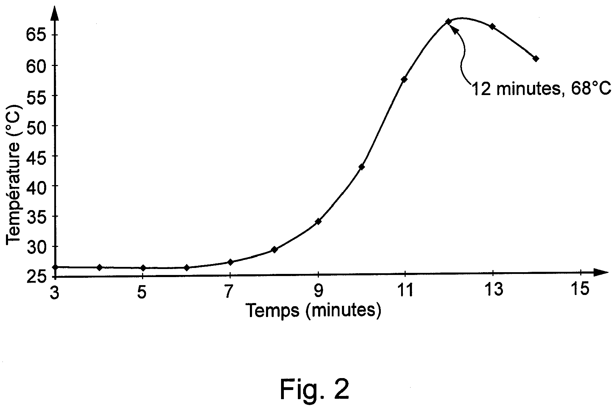

Les Figures 1 et 2 représentent non limitativement des dispositifs selon l'invention.

Un dispositif selon l'invention est représenté, à titre d'exemple non limitatif sur la Figure 1-A, sur laquelle est représentée une seringue (1) mono-compartiment (en position bloquée) contenant une formulation monophasique (2), un élément (3) comprenant un support sur lequel est déposé un film contenant l'amorceur, ledit élément étant connecté à un embout (4). L'élément (3) peut être un mélangeur statique. A titre d'exemple sur la Figure 1-A est présenté un mélangeur statique à ailettes, lesdites ailettes étant revêtues d'un film d'amorceur représenté par des stries, Le mélangeur statique peut également être de type différent.

Le volume de la formulation monophasique conservé dans le compartiment du système d'injection est choisi tel qu'il soit supérieur au volume nécessaire (Vutile) pour fixer la prothèse ou pour réaliser le comblement osseux additionné des volumes de l'élément contenant l'amorceur (Vm) et de l'injecteur (embout simple, canule ou trocart) (Vi), à savoir :

$$\text{Volume formulation monophasique} > (\text{Vutile} + \text{Vm} + \text{Vi}),$$

La pré-polymérisation du ciment est déclenchée lors de l'étape d'activation/maturation des formulations monophasiques par leur mise en contact avec le film contenant l'amorceur déposé sur les parois d'un élément du dispositif. Le film d'amorceur se solubilise rapidement dans la formulation. Les radicaux primaires amorceurs sont générés rapidement par action de l'activateur contenu dans les formulations monophasiques initiant dès la température ambiante la polymérisation. La concentration en amorceur ainsi que les caractéristiques du polymère filmifiant, typiquement du PMMA ou un de ses copolymères, déterminent la vitesse de dissolution de l'amorceur ainsi que la quantité et la distribution des radicaux au sein de la formulation. La polymérisation radicalaire s'effectue alors selon une cinétique et un dégagement de chaleur similaire à ceux des systèmes commerciaux bi- ou multiphasiques. Selon la proportion d'amorceur et d'activateur, on peut ajuster le temps de maturation/prépolymérisation, après quoi l'injection du système prépolymérisé dans la cavité osseuse ou dans la vertèbre peut être réalisée directement via l'injecteur connecté. La prise finale du ciment, correspondant à la fin de la phase de polymérisation, s'accompagne durant environ 2 à 5 minutes d'un exotherme de polymérisation conduisant à des températures maximales de 70 à 90°C au sein des ciments et dans les tissus biologiques situés à proximité immédiate, après quoi la température décroit avec l'achèvement de la polymérisation. Les taux résiduels en monomère mesurés dans les ciments sont inférieurs à 1% massique.

Le procédé d'activation/polymérisation des formulations monophasiques liquides et leur mode d'injection lors de l'intervention chirurgicale sont illustrés sur les Figures 1-B et 1-C.

La Figure 1-B représente l'étape de mélangeage/activation et de pré-polymérisation, au cours de laquelle la formulation monophasique liquide est mise en contact avec l'amorceur qui se trouve ainsi solubilisé (5)

La Figure 1-C représente l'étape d'injection, au cours de laquelle le ciment pré-polymérisé (6) est injecté dans la cavité osseuse (7).

L'invention concerne également un procédé d'injection de ciments polymères pour la chirurgie réparatrice ou de reconstruction osseuse, en particulier pour la fixation de prothèses, la réparation osseuse ou la vertébroplastie, ledit ciment étant préparé à partir d'une formulation monophasique liquide telle que décrite ci-dessus, dans lequel l'activation de la formulation monophasique est réalisée lors de l'opération d'injection sans nécessiter une opération de mélangeage manuel.

Les ciments obtenus à partir de ces formulations monophasiques, qui sont également un objet de l'invention, ont des propriétés physiques modulables en fonction de leur composition et notamment de la nature des monomères et polymères utilisés. On peut ainsi moduler la rigidité des ciments, et plus précisément l'abaisser soit en copolymérisant le ou les monomère(s) de la solution ou suspension avec des comonomères de type acrylate (acrylate de buyle, acrylate de 2-butylhexyle, par exemple), soit en introduisant des copolymères de type MMA avec des acrylates ou encore avec des polymères de basse Tg. Selon la composition des formulations multiphasiques, et par comparaison avec des essais réalisés avec un ciment commercial biphasique de référence (Biomet®), on peut abaisser le module de Young (rigidité) des ciments d'un facteur 2 à 10 et se rapprocher ainsi des valeurs de module d'Young proche de l'os, et en particulier de l'os spongieux vertébral humain.

Une alternative pour moduler les propriétés mécaniques des ciments issus de compositions monophasiques consiste à utiliser des polymères ou (co)polymères de plus faible masse molaire ou des mélanges de polymère ou (co)polymères de différentes masses molaires.

Une autre voie pour moduler les caractéristiques des ciments consiste à générer, à partir d'une autre formulation monophasique, un matériau de morphologie biphasique par incorporation d'un copolymère à blocs ayant un bloc de basse Tg non miscible dans la matrice polymère rigide. Un des copolymères les plus appropriés est un copolymère à blocs ayant un bloc de MMA ou riche en MMA et un bloc à base de monomères acrylates (alkyl acrylates). Ces ciments

présent des comportements mécaniques permettant d'absorber les chocs et de limiter la propagation de fractures.

Le procédé de préparation de ciments polymères à partir de formulations monophasiques liquides, tel que décrit ci-dessus, ainsi que le dispositif et le procédé d'application par activation/injection et les ciments préparés à partir de ces formulations monophasiques, selon l'invention, apportent des améliorations substantielles à la fois dans la préparation et dans les conditions de conservation sur des temps longs (3 ans) de formulations monophasiques pour ciments polymères pour applications en chirurgie réparatrice.

La présente invention permet notamment d'éviter les risques liés à la décomposition en radicaux potentiellement amorceurs, qui limite la conservation et l'utilisation dans le temps de ces solutions dont la viscosité augmente même lorsqu'elles sont conservées au réfrigérateur (4°C), ainsi que ceux liés à la maîtrise difficile de l'injection de deux solutions possédant des viscosités différentes à l'aide d'une seringue bi-compartiment. De plus, elle est d'une application beaucoup plus aisée pour le praticien.

Les formulations monophasiques liquides selon l'invention peuvent être conservées dans un dispositif comprenant un système d'injection à un compartiment et un élément de mise en contact comprenant un support sur lequel est déposé(e) la couche mince (ou film) contenant l'amorceur, pour être injectées directement dans la cavité osseuse ou la vertèbre du patient. La mise en oeuvre du ciment, à savoir l'activation de la formulation monophasique lors de l'opération d'injection est réalisée, en condition étanche, dans le dispositif, sans nécessiter une opération de mélangeage manuel.

Par rapport aux procédés existants, l'invention apporte des solutions nouvelles et simplifiées dans le procédé de mélangeage/activation/injection de ces formulations monophasiques. De plus, les ciments issus des formulations monophasiques liquides décrites ci-dessus présentent des propriétés en compression, flexion, cisaillement permettant de réduire les risques de fracture des parties osseuses et des vertèbres traitées et ou voisines.

Les exemples suivants permettent d'expliciter l'invention concernant la préparation de formulations monophasiques et leur procédé d'activation/injection en tant que ciments pour les chirurgies réparatrices, de reconstruction, ou esthétique. Ces exemples sont présentés à titre illustratif, i) du procédé de préparation des formulations monophasiques liquides, ii) des caractéristiques mécaniques des ciments obtenus par le procédé et iii) des applications du procédé dans le domaine biomédical, mais aucunement à titre exclusif et limitatif.

Exemple 1: Préparation de formulations monophasiques selon l'invention contenant un monomère et un activateur (DMPT) ainsi qu'un polymère et un agent radio-opacifiant

Les formulations 1-a) à 1-e) ont été préparées comme décrit ci-dessous. L'évolution de leur viscosité a été mesurée au cours du temps par rhéométrie.

Formulation 1-a)

31 g de copolymère statistique PMMA-MABu, (Degalan LP6311, masse molaire 30000 g/mol) sont solubilisés dans 10 g MMA. Après solubilisation 22 g d'oxyde de zirconium (Minchen UK DKK Japon) et 0,145 g de N,N-diméthyl, para-toluidine, sont ensuite ajoutés sous agitation.

La formulation 1-a) une fois homogénéisée est placée dans le noir à 20°C et l'évolution de sa viscosité est mesurée par rhéomètrie au cours du temps. La viscosité varie de 100 Pa.s à 1300Pa.s au bout de quelques semaines.

Formulation 1-b)

200 ppm d'hydroquinone sont ajoutés, en tant que capteurs de radicaux libre amorceurs, à la formulation a) et l'évolution de la viscosité au cours du temps de la formulation (conservée à 20°C dans le noir) est suivie par rhéomètre. La viscosité varie de 100 Pa.s à 800 Pa.s au bout de trois mois.

Formulation 1-c)

Dans la formulation 1-a), le PMMA, (Degalan LP6311, masse molaire 30 000 g/mol) est préalablement traité à 120°C pendant 24 h avant d'être solubilisé dans le MMA. La formulation correspondante c) obtenue, une fois homogénéisée est placée dans le noir à 20°C et l'évolution de sa viscosité est mesurée par rhéomètrie au cours du temps. La viscosité varie de 100 Pa.s à 400 Pa.s au bout d'un an.

Formulation 1-d)

Dans la formulation 1-a), le PMMA, (Degalan LP6311, masse molaire 30 000 g/mol) est préalablement traité à 120°C pendant 24 h avant d'être solubilisé dans le MMA. 200 ppm d'hydroquinone sont également ajoutés, en tant que capteurs de radicaux libres. La formulation 1-d), une fois homogénéisée est placée dans le noir à 20°C et l'évolution de sa viscosité est mesurée par rhéométrie au cours du temps. La viscosité varie de 100 Pa.s à 300 Pa.s au bout de 3 ans.

L'activation et la mise en oeuvre des formulations correspondantes selon le protocole opérationnel de l'invention montrent que, après différents temps de stockage, les cinétiques de polymérisation et les caractéristiques mécaniques des ciments n'ont subi aucune modification significative et présentent les critères requis.

Formulation 1-e)

Le polymère PMMA, 14 g, (Degalan 6606F, masse molaire 300000 g/mol) a été préalablement traité pendant 24 h à 120°C avant d'être solubilisé dans 42 g de MMA. Après solubilisation 44 g d'oxyde de zirconium (Minchen UK DKK Japon), et 100 ppm d'un capteur de radicaux libres, l'hydroquinone, sont ensuite ajoutés sous agitation. La formulation une fois homogénéisée est placée dans le noir à 20°C.

Exemple comparatif 2 : solutions contenant le monomère et l'amorceur (correspondant au procédé dit multi-solutions)

Cet exemple illustre l'évolution au cours du temps, à 20°C, de la viscosité d'une solution contenant à la fois le monomère (MMA), l'amorceur (BPO) ainsi qu'un polymère, un agent radio-opacifiant et éventuellement un capteur de radicaux libres. Le copolymère statistique PMMA-MABu, 31 g, (Degalan LP6311, masse molaire 30000 g/mol) a été préalablement traité pendant 24 h à 120°C avant d'être solubilisé dans 10 g de MMA. Après solubilisation 31 g d'oxyde de zirconium (Minchen UK DKK Japon), 0,25 g de peroxyde de benzoyle (BPO), et 200 ppm d'un capteur de radicaux libres, l'hydroquinone, sont ensuite ajoutés sous agitation. La formulation une fois homogénéisée est placée dans le noir à 20°C et l'évolution de sa viscosité est mesurée par rhéométre au cours du temps. La viscosité varie de 100 Pa.s à 30000 Pa.s au bout de 7 mois.

Cet exemple montre que les formulations monophasiques selon l'invention présentent des propriétés avantageuses de conservation dans le temps contrairement aux formulations « multi-solutions », dont la viscosité augmente de manière importante au cours du temps.

Exemple 3: Préparation de formulations monophasiques selon l'invention

3.1) Formulation à base de PMMA et de MMA.

10 g de polyméthacrylate de méthyle (PMMA, Degalan M825, de masse molaire 80000 g/mol) prétraité à 120°C est solubilisé dans 10 g de MMA. Est ensuite ajouté au mélange 16,02 g d'oxyde de zirconium (Minchen UK DKK Japon) de granulométrie 0,53 $\mu$m 0,145g de N,N-diméthyl,*para*-toluidine, et enfin 200 ppm d'hydroquinone. La formulation monophasique présente une viscosité initiale déterminée par rhéométrie de l'ordre de 1000 Pa.s, à 20°C.

3.2) Formulation à base d'un copolymère à blocs PMMA-b-MABu et de MMA.

31 g d'un copolymère à bloc poly(méthacrylate de méthyle-*bloc*-méthacrylate de butyle) (PMMA-*b*-MaBu, préparé par polymérisation radicalaire contrôlée, de masse molaire apparente 30000 g/mol) et prétraité à 120°C est solubilisé dans 10 g de MMA. Est ensuite ajouté au mélange 22 g d'oxyde de zirconium (Minchen UK DKK Japon) de granulométrie 0,53 $\mu$m, 0,145 g de N,N-diméthyl,para-toluidine, et enfin 200 ppm d'hydroquinone. La formulation monophasique présente une viscosité initiale de l'ordre de 100 Pa.s, à 20°C.

Exemple 4: Préparation et application de ciments polymères (activation/iniection)

4.1) Revêtement d'un élément du dispositif par un film d'amorceur : exemple d'un mélangeur à ailettes

Les ailettes internes d'un mélangeur statique sont plongées dans une solution d'acétone et de BPO purifié (50% massique), afin d'en revêtir leur surface. Le solvant est ensuite éliminé par évaporation et séchage sous vide, laissant un film homogène de BPO sur les parois des ailettes du mélangeur. La quantité de BPO déposée, déterminée par augmentation de masse du mélangeur, peut être ajustée en jouant sur la concentration de la solution. Typiquement celles-ci contiennent entre 30 et 70 % de BPO.

Une alternative consiste à utiliser une solution d'acétone et d'amorceur avec un polymère ou un copolymère filmifiant. On peut par exemple utiliser des copolymères p(MMA-MABu) de composition proche de celle utilisée dans les formulations monophasiques. Ainsi dans le cas d'une solution à 50% massique de copolymère p(MMA-MaBu) (Degalan LP6311, masse molaire 30 000 g/mol) et de BPO (proportion massique polymère : amorceur 1 :2) dans l'acétone, la quantité de BPO déposée est de l'ordre de 200 mg, quantité largement suffisante pour obtenir des cinétiques

rapides de polymérisation.

<u>4.2) Activation/pré-polymérisation</u>

On utilise un dispositif tel que représenté sur la Figure 1. 10 cm$^3$ de la formulation monophasique (formulations 1-a) à 1-e) de l'exemple 1) contenue dans la chambre de la seringue (Figure 1-A) sont injectés lentement dans un élément du dispositif constitué par un mélangeur statique équipé d'ailettes sur les parois duquel un film contenant 200 mg d'amorceur a été déposé (Figure 1-B). Lors du remplissage du mélangeur par la formulation monophasique et de sa mise en contact avec le film de BPO déposé, celui-ci est rapidement solubilisé (2 min) permettant l'interaction entre le BPO et la N,N diméthylamino *para* toluidine et le démarrage rapide, dés la température ambiante, de la polymérisation des monomères acryliques. Celle-ci se déroule alors selon une cinétique semblable à celle observée dans le cas des formulations bi- ou poly-phasiques. En fonction de la proportion d'amorceur et d'activateur introduit, le maximum de l'exotherme de polymérisation varie de 65 à 85°C. Celui-ci est atteint entre 10 à 15 min après la mise en contact de la formulation avec le film d'amorceur.

La courbe de l'augmentation de la température en fonction du temps observé après injection de la formulation 1-b) de l'exemple 1 est représentée sur la Figure 2.

<u>4.3) Injection</u>

La formulation activée par passage sur le film de polymère contenant l'amorceur est injectée directement par l'intermédiaire d'un embout simple, d'une canule, ou d'un trocart, dans la cavité osseuse où doit être fixée la prothèse (dans l'os ou la vertèbre à réparer ou à reconstruire).

<u>4.4) Propriétés mécaniques</u>

Les ciments obtenus avec les formulations testées peuvent présenter soit des modules plus proches de ceux des os spongieux vertébraux humains soit des modules équivalents aux modules des ciments obtenus avec des produits commerciaux.

Les propriétés mécaniques des formulations selon l'invention ont été mesurées selon la norme ISO 5833.

Les résultats sont rapportés dans le tableau 1 ci-dessous :

Tableau 1

| Formulation utilisée | Rapport massique BPO/PMMA | Masse BPO déposée (mg) | n | $\sigma_{moy}$ (décalage 2%) MPa | $E_{moy}$ (MPa) |
|---|---|---|---|---|---|
| Formulations 1-a) à 1-d) de l'exemple 1 | 4 | 200 | 20 | 21,9 $\pm$ 4,0 | 910$\pm$80 |
| Formulation 1-e) de l'exemple 1 | 4 | 200 | 6 | 80$\pm$ 5,0 | 1870 $\pm$ 352 |
| Os spongieux vertébral humain | Données littérature | | / | 2,1 $\pm$ 1,8 | 187 $\pm$ 143 |
| Ciment osseux commercial (type Biomet Bone Cement V) | Essais réalisés dans des conditions identiques | | 15 | 72 $\pm$ 4,2 | 1665 $\pm$ 327 |
| n = nombre d'essais réalisés $\sigma_{moy}$ = résistance moyenne à la compression $E_{moy}$ = module de flexion | | | | | |

Les résultats montrent que les propriétés mécaniques des formulations 1-a) à 1-d) sont particulièrement adaptées à la vertébroplastie alors que la formulation 1-e) qui répond à la norme ISO5833 peut être utilisée sans restriction dans le domaine des interventions orthopédiques (hanche, genou, etc....).

**Revendications**

**1.** Procédé de préparation de ciments polymères pour la chirurgie réparatrice ou de reconstruction osseuse, comprenant la mise en contact d'une formulation monophasique liquide, ladite formulation comprenant une phase liquide contenant au moins un polymère ou copolymère en solution ou en suspension dans au moins un monomère, et au moins un activateur de polymérisation, avec un amorceur de polymérisation radicalaire déposé sous forme de couche mince ou de film sur un support.

**2.** Procédé selon la revendication 1, **caractérisé en ce que** la mise en contact de ladite formulation monophasique liquide avec un amorceur de polymérisation radicalaire déposé sous forme de couche mince ou de film sur un support, permet la solubilisation de ladite couche mince ou film contenant l'amorceur, la mise en contact de l'amorceur et de l'activateur de polymérisation radicalaire et l'amorçage de la polymérisation du ou des monomères.

**3.** Procédé selon la revendication 1 ou 2, **caractérisé en ce que** la mise en contact de ladite formulation monophasique liquide avec au moins un amorceur de polymérisation radicalaire est effectuée par le passage de ladite formulation monophasique liquide sur un élément comprenant un support sur lequel est déposé(e) une couche mince ou un film contenant un amorceur de polymérisation radicalaire.

**4.** Procédé selon l'une quelconque des revendications 1 à 3, **caractérisé en ce que** la mise en contact de ladite formulation monophasique liquide avec au moins un amorceur de polymérisation radicalaire est réalisée dans un dispositif d'injection dans lequel ladite formulation monophasique liquide est contenue dans le réservoir unique d'un dispositif d'injection équipé en sortie d'un élément comprenant un support sur lequel est déposé(e) une couche mince ou un film contenant l'amorceur de polymérisation.

**5.** Procédé selon l'une quelconque des revendications 1 à 4, **caractérisé en ce que** la mise en contact de ladite formulation monophasique liquide, avec un amorceur de polymérisation radicalaire déposé sous forme de couche mince ou de film sur un support a lieu au moment de l'intervention chirurgicale.

**6.** Procédé selon l'une quelconque des revendications 1 à 6, **caractérisé en ce que** ladite formulation monophasique liquide contient au moins un agent radioopacifiant.

**7.** Procédé selon l'une quelconque des revendications 1 à 7, **caractérisé en ce que** la formulation monophasique liquide contient au moins un monomère acrylique, de préférence (méth)acrylique, un ou plusieurs polymères ou copolymères, au moins un activateur de polymérisation radicalaire, au moins un agent radio-opacifiant et au moins un additif.

**8.** Procédé selon l'une des revendications 1 à 7, **caractérisé en ce que** ledit additif est choisi parmi les inhibiteurs de radicaux libres et les antibiotiques.

**9.** Procédé selon l'une quelconque des revendications 1 à 8, **caractérisé en ce que** le(s) polymère(s) ou le(s) copolymère(s) sont choisi(s) parmi le poly(méthacrylate de méthyle) et le(s) copolymère(s) statistique(s) ou à blocs de méthacrylate de méthyle avec un monomère de type acrylate ou méthacrylate.

**10.** Procédé selon l'une quelconque des revendications 1 à 9, **caractérisé en ce que** les chaînes du polymère ou du copolymère ont une structure linéaire, ramifiée ou hyper-ramifiée, ou encore forment des particules constituées d'un ensemble de chaînes réticulées ou non réticulées.

**11.** Procédé selon l'une quelconque des revendications 1 à 10, **caractérisé en ce que** le polymère ou le copolymère est de type à blocs et possède au moins un bloc non miscible avec les autres polymères et copolymères et dont le bloc non miscible a une température de transition vitreuse inférieure à 35°C.

**12.** Procédé selon l'une quelconque des revendications 1 à 11, **caractérisé en ce que** le polymère ou le copolymère est traité thermiquement à 120°C pendant plusieurs heures avant d'être introduits dans la formulation.

**13.** Procédé selon l'une quelconque des revendications 1 à 12, **caractérisé en ce que** le film déposé sur le support et contenant l'amorceur de polymérisation radicalaire est constitué par un mélange de l'amorceur et d'un polymère ou copolymère filmifiant.

**14.** Procédé selon l'une des revendications 1 à 13, **caractérisé en ce qu'**il comprend une étape de pré-polymérisation de la formulation monophasique dans un élément d'un dispositif d'injection comprenant un support sur lequel est déposé(e) une couche mince (ou film) contenant un amorceur de polymérisation , réalisée par activation de l'amorceur de polymérisation par l'activateur contenu dans la formulation monophasique.

15. Ciments polymères pour la chirurgie réparatrice ou de reconstruction osseuse à base de formulations monophasiques liquides dont la rigidité est modulée soit par l'utilisation de copolymères de faibles masse molaire ou de mélanges de (co)polymères de différentes masses, soit par incorporation d'un copolymère à blocs ayant un bloc ayant une basse Tg non miscible dans la matrice polymère rigide.

16. Dispositif pour l'injection d'un ciment polymère pour la chirurgie réparatrice ou de reconstruction osseuse, comprenant

- un système d'injection à un compartiment contenant la formulation monophasique liquide comprenant une phase liquide contenant au moins un polymère ou copolymère en solution ou en suspension dans au moins un monomère, et au moins un activateur de polymérisation radicalaire, décrite dans l'une quelconque des revendications 1 à 12,
- un élément comprenant un support sur lequel est déposé(e) une couche mince ou un film contenant un amorceur de polymérisation.

17. Dispositif selon la revendication 16, **caractérisé en ce qu'**il est connecté, à un système injecteur permettant d'injecter directement le ciment pré-polymérisé, en cours de prise, au site de traitement.

18. Dispositif selon les revendications 16 ou 17, **caractérisé en ce que** le système d'injection à un compartiment contenant la formulation monophasique liquide, un élément comprenant un support sur lequel est déposé(e) une couche mince ou un film contenant un amorceur de polymérisation, et, de manière optionnelle, le système injecteur, sont conditionnés séparément, en vue d'une utilisation conjointe.


**Patentansprüche**

1. Verfahren zur Herstellung von Polymerzementen für die wiederherstellende oder rekonstruktive Knochenchirurgie, welches das Inkontaktbringen einer einphasigen flüssigen Formulierung, wobei die Formulierung eine flüssige Phase umfasst, welche zumindest ein Polymer oder Copolymer, aufgelöst oder suspendiert in zumindest einem Monomer, und zumindest einen Polymerisationsaktivator enthält, mit einem in Form einer Dünnschicht oder eines Films auf einem Träger aufgebrachten Initiator für radikalische Polymerisation umfasst.

2. Verfahren nach Anspruch 1, **dadurch gekennzeichnet, dass** das Inkontaktbringen der einphasigen flüssigen Formulierung mit einem Initiator für radikalische Polymerisation, der in Form einer Dünnschicht oder eines Films auf einem Träger aufgebracht ist, das Löslichmachen der Dünnschicht oder des Films, welche(r) den Initiator enthält, das Inkontaktbringen des Initiators und des Aktivators für radikalische Polymerisation und die Initiierung der Polymerisation des Monomers oder der Monomere ermöglicht.

3. Verfahren nach Anspruch 1 oder 2, **dadurch gekennzeichnet, dass** das Inkontaktbringen der einphasigen flüssigen Formulierung mit zumindest einem Initiator für radikalische Polymerisation dadurch erfolgt, dass die einphasige flüssige Formulierung auf ein Bauteil gelangt, das einen Träger umfasst, auf dem eine Dünnschicht oder ein Film aufgebracht ist, welche(r) einen Initiator für radikalische Polymerisation enthält.

4. Verfahren nach einem der Ansprüche 1 bis 3, **dadurch gekennzeichnet, dass** das Inkontaktbringen der einphasigen flüssigen Formulierung mit zumindest einem Initiator für radikalische Polymerisation in einer Injektionsvorrichtung stattfindet, bei der die einphasige flüssige Formulierung in dem Einzelbehälter einer Injektionsvorrichtung enthalten ist, die ausgangsseitig mit einem Bauteil ausgestattet ist, das einen Träger umfasst, auf dem eine Dünnschicht oder ein Film aufgebracht ist, welche(r) den Polymerisationsinitiator enthält.

5. Verfahren nach einem der Ansprüche 1 bis 4, **dadurch gekennzeichnet, dass** das Inkontaktbringen der einphasigen flüssigen Formulierung mit einem Initiator für radikalische Polymerisation, der in Form einer Dünnschicht oder eines Films auf einem Träger aufgebracht ist, zum Zeitpunkt des chirurgischen Eingriffs stattfindet.

6. Verfahren nach einem der Ansprüche 1 bis 6, **dadurch gekennzeichnet, dass** die einphasige flüssige Formulierung zumindest ein Röntgen-Opazifizierungsmittel enthält.

7. Verfahren nach einem der Ansprüche 1 bis 7, **dadurch gekennzeichnet, dass** die einphasige flüssige Formulierung zumindest ein acrylisches, vorzugsweise (meth)acrylisches Monomer, ein oder mehrere Polymere oder Copolymere, zumindest einen Aktivator für radikalische Polymerisation, zumindest ein Röntgen-Opazifierungsmittel und zumindest ein Additiv enthält.

8. Verfahren nach einem der Ansprüche 1 bis 7, **dadurch gekennzeichnet, dass** das Additiv aus Inhibitoren für freie Radikale und Antibiotika ausgewählt wird.

9. Verfahren nach einem der Ansprüche 1 bis 8, **dadurch gekennzeichnet, dass** das/die Polymer(e) oder das/die Copolymer(e) aus Polymethylmethacrylat und einem bzw. mehreren statistischen Copolymer(en) oder Block-copolymer(en) aus Methylmethacrylat mit einem Acrylat- oder Methacrylatmonomer ausgewählt wird/werden.

10. Verfahren nach einem der Ansprüche 1 bis 9, **dadurch gekennzeichnet, dass** die Polymer- oder Copolymerketten eine lineare, verzweigte oder hyperverzweigte Struktur aufweisen, oder zusätzlich Partikel bilden, die aus einem Gefüge von vernetzten oder nicht vernetzten Ketten bestehen.

11. Verfahren nach einem der Ansprüche 1 bis 10, **dadurch gekennzeichnet, dass** das Polymer oder das Copolymer ein Blockpolymer oder -copolymer ist und zumindest einen Block besitzt, der mit den anderen Polymeren und Copolymeren nicht mischbar ist und dessen nicht mischbarer Block eine Glasübergangstemperatur von weniger als 35°C aufweist.

12. Verfahren nach einem der Ansprüche 1 bis 11, **dadurch gekennzeichnet, dass** das Polymer oder das Copolymer vor seiner Einarbeitung in die Formulierung mehrere Stunden bei 120°C wärmebehandelt wird.

13. Verfahren nach einem der Ansprüche 1 bis 12, **dadurch gekennzeichnet, dass** der Film, der auf dem Träger aufgebracht ist und den Initiator für radikalische Polymerisation enthält, aus einem Gemisch aus dem Initiator und einem filmbildenden Polymer oder Copolymer besteht.

14. Verfahren nach einem der Ansprüche 1 bis 13, **dadurch gekennzeichnet, dass** dieses einen Schritt der Vorpolymerisation der einphasigen Formulierung in einem Bauteil einer Injektionsvorrichtung umfasst, das einen Träger umfasst, auf dem eine Dünnschicht (oder ein Film) aufgebracht ist, welche(r) einen Polymerisationsinitiator enthält, wobei dieser Schritt mittels einer Aktivierung des Polymerisationsinitiators durch den in der einphasigen Formulierung enthaltenen Aktivator erfolgt.

15. Polymerzemente für die wiederherstellende oder rekonstruktive Knochenchirurgie auf der Basis einphasiger flüssiger Formulierungen, deren Steifigkeit entweder durch den Einsatz von Copolymeren mit geringem Molargewicht oder von Polymer- oder Copolymermischungen mit unterschiedlichem Gewicht, oder aber durch Einarbeitung eines Blockcopolymers mit einem nicht mischbaren Block mit einer niedrigen Tg in die steife Polymermatrix, verändert wird.

16. Vorrichtung zur Injektion eines Polymerzements für die wiederherstellende oder rekonstruktive Knochenchirurgie, welche Folgendes umfasst:

    - ein Einkammer-Injektionssystem, das die einphasige flüssige Formulierung enthält, die eine flüssige Phase umfasst, welche zumindest ein Polymer oder Copolymer, das in zumindest einem Monomer aufgelöst oder suspendiert ist, und zumindest einen Aktivator für radikalische Polymerisation, die in einem der Ansprüche 1 bis 12 beschrieben ist, enthält,
    - ein Bauteil, das einen Träger umfasst, auf dem eine Dünnschicht oder ein Film aufgebracht ist, welche(r) einen Polymerisationsinitiator enthält.

17. Vorrichtung nach Anspruch 16, **dadurch gekennzeichnet, dass** diese mit einem Injektorsystem verbunden ist, mit dem der vorpolymerisierte Zement während des Abbindens unmittelbar an der Behandlungsstelle eingespritzt werden kann.

18. Vorrichtung nach einem der Ansprüche 16 oder 17, **dadurch gekennzeichnet, dass** das Einkammer-Injektionssystem, das die einphasige flüssige Formulierung enthält, ein Bauteil, das einen Träger umfasst, auf dem eine Dünnschicht oder ein Film aufgebracht ist, welche(r) einen Polymerisationsinitiator enthält, und optional auch das Injektorsystem im Hinblick auf einen gemeinsamen Einsatz getrennt verpackt sind.

**Claims**

1. Method for preparing polymer cements for reparative surgery or for bone reconstruction, comprising bringing a liquid single-phase formulation, said formulation comprising a liquid phase containing at least one polymer or copolymer in solution or in suspension in at least one monomer, and at least one activator of polymerization, into contact with an initiator of radical polymerization deposited in the form of a thin layer or film on a support.

2. Method according to claim 1, **characterized in that** bringing said liquid single-phase formulation into contact with an initiator of radical polymerization deposited in the form of a thin layer or film on a support allows solubilization of said thin layer or film containing the initiator, bringing into contact the initiator and the activator of radical polymerization and initiation of polymerization of the monomer or monomers.

3. Method according to claim 1 or 2, **characterized in that** bringing said liquid single-phase formulation into contact with at least one initiator of radical polymerization is carried out by the passage of said liquid single-phase formulation over an element comprising a support, on which a thin layer or film containing an initiator of radical polymerization is deposited.

4. Method according to any one of claims 1 to 3, **characterized in that** bringing said liquid single-phase formulation into contact with at least one initiator of radical polymerization is carried out in an injection device in which said liquid single-phase formulation is contained in the single reservoir of an injection device equipped at the outlet with an element comprising a support on which a thin layer or a film containing the polymerization initiator is deposited.

5. Method according to any one of claims 1 to 4, **characterized in that** bringing said liquid single-phase formulation into contact with an initiator of radical polymerization deposited in the form of a thin layer or film on a support takes place at the moment of the surgical procedure.

6. Method according to any one of claims 1 to 6, **characterized in that** said liquid single-phase formulation contains at least one radio-opacifying agent.

7. Method according to any one of claims 1 to 7, **characterized in that** the liquid single-phase formulation contains at least one acrylic, preferably (meth)acrylic, monomer, one or more polymers or copolymers, at least one activator of radical polymerization, at least one radio-opacifying agent and at least one additive.

8. Method according to one of claims 1 to 7, **characterized in that** said additive is selected from free radical inhibitors and antibiotics.

9. Method according to any one of claims 1 to 8, **characterized in that** the polymer(s) or the copolymer(s) is/are selected from poly(methyl methacrylate) and random or block copolymer(s) of methyl methacrylate with a monomer of the acrylate or methacrylate type.

10. Method according to any one of claims 1 to 9, **characterized in that** the polymer or copolymer chains have a linear, branched or hyper-branched structure, or else form particles constituted by an assemblage of crosslinked or non-crosslinked chains.

11. Method according to any one of claims 1 to 10, **characterized in that** the polymer or copolymer is of the block type and has at least one block that is immiscible with the other polymers and copolymers and the immiscible block of which has a glass transition temperature below 35°C.

12. Method according to any one of claims 1 to 11, **characterized in that** the polymer or copolymer is treated thermally at 120°C for several hours before being introduced into the formulation.

13. Method according to any one of claims 1 to 12, **characterized in that** the film deposited on the support and containing the initiator of radical polymerization consists of a mixture of the initiator and of a film-forming polymer or copolymer.

14. Method according to one of claims 1 to 13, **characterized in that** it comprises a step of prepolymerization of the single-phase formulation in an element of an injection device comprising a support on which a thin layer (or film) containing a polymerization initiator is deposited, carried out by activation of the polymerization initiator by the activator contained in the single-phase formulation.

**15.** Polymer cements for reparative surgery or for bone reconstruction based on liquid single-phase formulations the rigidity of which is modulated either by using copolymers of low molecular weight or mixtures of (co)polymers of different molecular weights, or by incorporation of a block copolymer having a block having a low Tg that is immiscible in the rigid polymer matrix.

**16.** Device for injecting a polymer cement for reparative surgery or for bone reconstruction, comprising

- a one-compartment injection system containing the liquid single-phase formulation comprising a liquid phase containing at least one polymer or copolymer in solution or in suspension in at least one monomer, and at least one activator of radical polymerization, described in any one of claims 1 to 12,
- an element comprising a support on which a thin layer or a film containing a polymerization initiator is deposited.

**17.** Device according to claim 16, **characterized in that** it is connected to an injector system allowing direct injection of the prepolymerized cement, in the course of setting, at the treatment site.

**18.** Device according to claims 16 or 17, **characterized in that** the one-compartment injection system containing the liquid single-phase formulation, an element comprising a support on which a thin layer or a film containing a polymerization initiator is deposited, and, optionally, the injector system, are packaged separately, with a view to joint use.

Fig. 1A

Fig. 1B

Fig. 1C

Fig. 2

## RÉFÉRENCES CITÉES DANS LA DESCRIPTION

**Documents brevets cités dans la description**

- WO 2010005442 A
- US 20070027230 A
- WO 2010115138 A
- WO 2011004355 A
- US 20100228358 A

**Littérature non-brevet citée dans la description**

- **J. CHAMLEY.** *J.Bone Joint Surg,* 1960, vol. 42B, 28
- **LEWIS G.** J Biomed Mater Res B: Appl. Biomater. l'Agence Française de Sécurité Sanitaire des produits de santé et la Food and Drug Administration, 2008, vol. 84B, 301
- **LEWIS G.** Appl Biomater. *J. Biomed. Mater Res B,* 2006, vol. 76B, 456-468
- **MC GRAW JK et al.** *J Vasc Interv Radiol,* 2003, vol. 14, S311-5
- **GALIBERT P et al.** *Neurochirurgie,* 1987, vol. 33, 166
- **WIXSON, R.L. et al.** *J. of Arthroplasty,* 1987, vol. 2 (2), 141-149
- **SIVARAM, S. et al.** *Polymer Bulletin,* 1980, vol. 3 (1-2), 27-35
- **GRADOS et al.** *Rheumatology,* 2000, vol. 39, 1410-4
- **CYTEVAL et al.** *AJR,* 1999, vol. 173, 1685-90